# EUROPÄISCHE PATENTSCHRIFT

(11) **EP 0 684 811 B1**
(45) Veröffentlichungstag und Bekanntmachung des Hinweises auf die Patenterteilung: **14.10.1998**
(21) Anmeldenummer: 94907544.4
(22) Anmeldetag: 11.02.1994
(51) Int. Cl.: A61K 7/48, A61K 31/22, A61K 31/23

(54) **VERWENDUNG VON DIGLYCERINESTERN GEGEN UNREINE HAUT**
USE OF DIGLYCERINE ESTERS AGAINST SKIN IMPURITIES
UTILISATION DES ESTERS DE DIGLYCERINE CONTRE LES IMPURETES DE LA PEAU

(30) Priorität: 19.02.1993 DE 4305069
(43) Veröffentlichungstag der Anmeldung: 06.12.1995
(73) Patentinhaber: Beiersdorf Aktiengesellschaft, 20245 Hamburg (DE); Solvay Alkali GmbH, 30173 Hannover (DE)
(72) Erfinder: DILLENBURG, Helmut, D-47495 Rheinberg (DE); JAKOBSON, Gerald, D-47495 Rheinberg (DE); KLIER, Manfred, D-20521 Aumühle (DE); SIEMANOWSKI, Werner, D-47495 Rheinberg (DE); TRAUPE, Bernd, D-2247 Hamburg (DE); UHLIG, Karl-Heinz, D-47802 Krefeld-Traar (DE); WOLF, Florian, D-20251 Hamburg (DE)
(86) Internationale Anmeldenummer: EP9400390
(87) Internationale Veröffentlichungsnummer: WO9418943

(56) Entgegenhaltungen:
- EP-A- 0 379 658
- WO-A-90/10441
- WO-A-91/12010
- DE-C- 4 100 490
- US-A- 5 231 087
- PATENT ABSTRACTS OF JAPAN vol. 14, no. 377 (C-748)(4320) & JP,A,02 138 107 (SUNSTAR INC) 28. Mai 1990
- PATENT ABSTRACTS OF JAPAN vol. 17, no. 158 (C-1041) & JP,A,04 321 626 (HISAMITSU PHARMACEUT CO INC) 11. November 1992

## Beschreibung

Die vorliegende Erfindung betrifft Wirkstoffe und Zubereitungen, solche Wirkstoffe enthaltend, welche gegen unreine Haut wirksam sind.

Bei der unreinen Haut sind neben anderen Einflüssen bakterielle Sekundärinfektionen von ätiologischer Bedeutung. Einer der wichtigsten Mikroorganismen, der in Zusammenhang mit unreiner Haut steht, ist *Propionibacterium acnes*.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.

Aufgabe der vorliegenden Erfindung war es also, einen gegen unreine Haut bzw. Propionibacterium acnes wirksamen Stoff zu finden.

Es wurde überraschend gefunden, und darin liegt die Lösung der Aufgabe, daß die Verwendung von Monocarbonsäureestern des Diglycerins zur Herstellung von Zubereitungen gegen unreine Haut und/oder gegen *Propionibacterium acnes*, sowie die Verwendung von Monocarbonsäureestern des Diglycerins zur Herstellung von Zubereitungen gegen unreine Haut und/oder gegen *Propionibacterium acnes* in kosmetischen Zubereitungen diese Aufgabe erfüllt.

Zwar werden in der WO-A-90/10441 gegen *Propionibacterium acnes* wirksame Triglycerinester beschrieben. Diese Schrift konnte allerdings nicht den Weg zur vorliegenden Erfindung weisen.

Erfindungsgemäß liegen die Diglycerineinheiten der erfindungsgemäßen Monocarbonsäureester als lineare, unverzweigte Moleküle, also über die jeweiligen OH-Gruppen in 1- bzw. 3-Stellung veretherte "Monoglycerinmoleküle" vor. 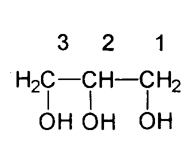 (Glycerin, mit Substitutionspositionen)

Ein geringer Anteil zyklischer Di- bzw. Triglycerineinheiten sowie über die OH-Gruppen in 2-Stellung veretherte Glycerinmoleküle kann geduldet werden. Es ist jedoch von Vorteil, solche Verunreinigungen so gering wie nur möglich zu halten.

Die erfindungsgemäßen Monocarbonsäureester des Diglycerins sind vorzugsweise Monocarbonsäuremonoester und bevorzugt durch folgende Struktur gekennzeichnet (Substitutionspositionen angegeben): 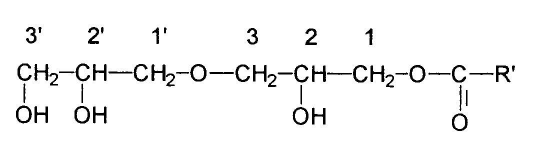 wobei R' einen Kohlenwasserstoffrest, vorteilhaft einen verzweigten oder unverzweigten Alkyl- oder Alkenylrest von 5 bis 17 C-Atomen darstellt.

Die diesen Estern zugrundeliegenden Säuren sind die

| | | |
|---|---|---|
| Hexansäure | (Capronsäure) | (R' = -C₅H₁₁), |
| Heptansäure | (Önanthsäure) | (R' = -C₆H₁₃), |
| Octansäure | (Caprylsäure) | (R' = -C₇H₁₅), |
| Nonansäure | (Pelargonsäure) | (R' = -C₈H₁₇), |
| Decansäure | (Caprinsäure) | (R' = -C₉H₁₉), |
| Undecansäure | | (R' = -C₁₀H₂₁), |
| 10-Undecensäure | (Undecylensäure) | (R' = -C₁₀H₁₉), |
| Dodecansäure | (Laurinsäure) | (R' = -C₁₁H₂₃), |
| Tridecansäure | | (R' = -C₁₂H₂₅), |
| Tetradecansäure | (Myristinsäure) | (R' = -C₁₃H₂₇), |
| Pentadecansäure | | (R' = -C₁₄H₂₉), |
| Hexadecansäure | (Palmitinsäure) | (R' = -C₁₅H₃₁), |
| Heptadecansäure | (Margarinsäure) | (R' = -C₁₆H₃₃), |
| Octadecansäure | (Stearinsäure) | (R' = -C₁₇H₃₅). |

Besonders günstig wird R' gewählt aus der Gruppe der unverzweigten Alkylreste mit ungeraden C-Zahlen, insbesondere mit 9, 11 und 13 C-Atomen.

Ganz besonders günstig sind

| | | |
|---|---|---|
| Diglycerinmonocaprinat | (DMC) | R' = 9 |
| Diglycerinmonolaurat | (DML) | R' = 11 |

Als bevorzugter erfindungsgemäßer Monocarbonsäureester hat sich das Diglycerinmonocaprinat (DMC) erwiesen.

Die erfindungsgemäßen Monofettsäureester des Diglycerins liegen bevorzugt in 1-Stellung, verestert vor.

Nach einer vorteilhatten Ausführungsform der vorliegenden Erfindung wird ein zusätzlicher Anteil an in anderen Stellen verestertem Diglycerin, ebenso wie gegebenenfalls ein Anteil an den verschiedenen Diestern des Diglycerins verwendet.

Insbesondere vorteilhaft sind solche Monocarbonsäureester, welche nach einem Verfahren erhältlich sind, wie es in der DE-OS 38 18 293 beschrieben wird.

Die Diglycerinester, welche sich durch zwei Asymmetriezentren auszeichnen, sind in all ihren Konfigurationen erfindungsgemäß wirksam. Die Diglycerinester besitzen vier Stereoisomere.

Bei den Diglycerinestern sind die 2- und die 2'-Position Asymmetriezentren. Erfindungsgemäß aktiv und gleichermaßen von Vorteil sind die 2S2'S-, die 2R2'S-, die 2S2'R- und die 2R2'R-Konfiguration.

Es hat sich als günstig herausgestellt, racemische Gemische der Stereoisomeren zu verwenden.

Nach einer vorteilhaften Ausführungsform der vorliegenden Erfindung werden Gemische eines oder mehrerer Monocarbonsäureester des Diglycerins mit einem oder mehreren Monocarbonsäureestern des Triglycerins verwendet.

Nach einer weiteren vorteilhaften Ausführungsform der vorliegenden Erfindung werden ein oder mehrere Monocarbonsäureester des Diglycerins und/oder ein oder mehrere Monocarbonsäureester des Triglycerins in Kombination mit anderen üblichen Wirkstoffen (Ersatzwirkstoffe), Hilfs-, Verschnitt- und/oder Zusatzstoffen eingesetzt.

Vorteilhaft liegen dann die Verschnittstoffe und/oder Ersatzwirkstoffe in einer Konzentration bis zu 50 Gew.-Teilen vor, bevorzugt bis zu 35 Gew.-Teilen, bezogen auf das 100 Gew.-Teile der Gesamtmenge, welche sich aus dem Monocarbonsäureester bzw. den Monocarbonsäureestern des Diglycerins und diesen Ersatzwirkstoffen und/oder Verschnittstoffen zusammensetzt.

Nach einer weiteren vorteilhatten Ausführungsform der vorliegenden Erfindung werden ein oder mehrere Monocarbonsäureester des Diglycerins in Kombination mit anderen gegen Acne vulgaris wirksamen oder das Wachstum von *Propionibacterium acnes* hemmend oder Propionibacterium acnes abtötend wirkenden Stoffen eingesetzt.

Nach noch einer weiteren vorteilhatten Ausführungsform der vorliegenden Erfindung werden ein oder mehrere Monocarbonsäureester des Diglycerins in Kombination mit Monocarbonsäureestern des Glycerins (also des "Monoglycerins") eingesetzt. Dabei übernehmen diese Monocarbonsäureester des Glycerins die Rolle der Verschnittstoffe und/oder Ersatzwirkstoffe und werden vorzugsweise in einer Konzentration bis zu 50 Gew.-Teilen, bevorzugt bis zu 35 Gew.-Teilen eingesetzt, bezogen auf 100 Gew.-Teile der Gesamtmenge, welche sich aus dem Monocarbonsäureester bzw. den Monocarbonsäureestern des Diglycerins und diesen Monocarbonsäureestern des Glycerins zusammensetzt.

Solche Monocarbonsäureester des Glycerins sind günstig gekennzeichnet durch eine Struktur wie folgt: 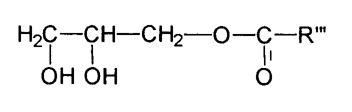 wobei R''' einen Kohlenwasserstoffrest, vorteilhaft einen verzweigten oder unverzweigten Alkyl- oder Alkenylrest von 5 bis 17 C-Atomen darstellt.

Erfindungsgemäß gegen unreine Haut wirksame Zubereitungen sind besonders vorteilhaft dadurch gekennzeichnet, daß der oder die Monocarbonsäureester des Diglycerins in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt oder vorliegen.

Erfindungsgemäß gegen unreine Haut wirksame Zubereitungen können in Form von mittels Pinseln oder Abstreifern oder Roll-on-Vorrichtungen auftragbaren flüssigen Zusammensetzungen, als Stifte und in Form von aus normalen Flaschen und Behältern auftragbaren W/O- oder O/W-Emulsionen, z.B. Cremes oder Lotionen. Weiterhin können erfindungsgemäß gegen unreine Haut wirksame Zubereitungen vorteilhaft in Form von Gesichtswässern, Tinkturen oder Reinigungsformulierungen vorliegen.

Als übliche Trägerstoffe zur Herstellung der erfindungsgemäß gegen unreine Haut wirksamen Zubereitungen können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroryethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosität.

Als Emulgatoren zur Herstellung der erfindungsgemäß gegen unreine Haut wirksamen Zubereitungen, welche vorteilhaft als flüssige Zubereitungen auf die gewünschten Hautbereiche aufgetragen werden sollen, vorteilhaft mittels eines Wattebausches, und die in den Zubereitungen in geringer Menge, z.B. 2 bis 5 Gewichts.-%, bezogen auf die Gesamt-Zusammensetzung, verwendet werden können, haben sich nichtionogene Typen, wie Polyoryethylen-Fettalkoholether, z.B. Cetostearylalkoholpolyethylenglykolether mit 12 bzw. 20 angelagerten Ethylenoxid-Einheiten pro Molekül, Cetostearylalkohol sowie Sorbitanester und Sorbitanester-Ethylenoxid-Verbindungen (z.B. Sorbitanmonostearat und Polyoryethylensorbitanmonostearat) und langkettige höhermolekulare wachsartige Polyglykolether als geeignet erwiesen.

Zusätzlich zu den genannten Bestandteilen können den gegen unreine Haut wirksamen Zubereitungen gemäß der Erfindung, deren pH-Wert vorzugsweise z.B. durch übliche Puffergemische auf 4,0 bis 9,0 insbesondere 5,0 bis 6,5, eingestellt wird, Parfüm, Farbstoffe, Antioxidantien (z.B. _-Tocopherol und seine Derivate oder Butylhydroxytoluol (BHT = 2,6-Di-Tert.-butyl-4-methylphenol) in Mengen von 0,01 bis 0,03 %, bezogen auf die Gesamtzusammensetzung), Suspendiermittel, Puffergemische oder andere übliche kosmetische Grundstoffe beigemischt werden.

Vorteilhaft wird der pH-Wert der erfindungsgemäß gegen unreine Haut wirksamen Zubereitungen im schwach sauren bis schwach alkalischen Bereich eingestellt, bevorzugt von 4,0 - 9,0, besonders bevorzugt von 5,0 - 6,5.

Die jeweils einzusetzenden Mengen an Trägerstoffen können in Abhängigkeit von der Art des jeweiligen Produktes vom Fachmann durch einfaches Ausprobieren leicht ermittelt werden.

Die Herstellung der Zubereitungen gemäß der Erfindung erfolgt, abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat, gegebenenfalls unter Erwärmen hergestellt und dann emulgiert.

Ansonsten sind die üblichen Maßregeln für das Zusammenstellen von galenischen Formulierungen zu beachten, die dem Fachmann geläufig sind.

Es folgen vorteilhafte Ausführungsbeispiele der vorliegenden Erfindung. Es werden für die erfindungsgemäßen Ester die vorbeschriebenen Abkürzungen verwendet.

### Beispiel 1

| Gel I, dünnflüssig | |
|---|---|
| (a) | Gew.-% |
| 1,3-Butylenglycol | 2,00 |
| Hydroxyethylcellulose (z.B. Tylose 4000, Hoechst) | 0,50 |

| (b) | |
|---|---|
| Ethylalkohol | 60,00 |
| PEG-40-Hydriertes Ricinusöl | 2,00 |
| DMC | 0,80 |

| (c) | |
|---|---|
| Wasser | ad 100,00 |

Die unter (a) genannten Bestandteile werden dispergiert, Wasser (c) wird zugegeben, bei Raumtemperatur quellen gelassen, eine Lösung der unter (b) genannten Bestandteile wird nach ca. 15 Minuten zugegeben. Die entstanden Mischung wird homogenisiert und kann abgefüllt werden.

### Beispiel 2

| Wachsstift I | |
|---|---|
| | Gew.-% |
| Hydriertes Ricinusöl | 5,00 |
| Bienenwachs | 6,00 |
| Ceresin (Hart-Ozokerit) | 30,00 |
| C₁₂₋₁₅-Alkyl-Benzoate | 17,00 |
| DMC | 0,90 |
| Octyldodecanol | ad 100,00 |

Die Bestandteile werden bei ca. 75° C aufgeschmolzen, gut vermischt und in geeignete Formen gegossen.

### Beispiel 3

| Wachsstift III | |
|---|---|
| | Gew.-% |
| Hydriertes Ricinusöl | 5,00 |
| Bienenwachs | 6,00 |
| Ceresin (Hart-Ozokerit) | 30,00 |
| C₁₂₋₁₅-Alkyl-Benzoate | 17,00 |
| DML | 1,45 |
| Octyldodecanol | ad 100,00 |

Die Bestandteile werden bei ca. 75° C aufgeschmolzen, gut vermischt und in geeignete Formen gegossen.

### Beispiel 4

| Reinigungsemulsion I | |
|---|---|
| (a) | Gew.-% |
| Tricetearethphosphat | 0,30 |
| Octyldodecanol | 2,00 |
| C₁₂₋₁₅-Alkyl-Benzoate | 2,00 |
| DMC | 0,50 |
| C₁₂₋₃₀-Alkylacrylate | 0,15 |

| (b) | |
|---|---|
| Ethylalkohol | 10,00 |

| (c) | |
|---|---|
| NaOH | 0,05 |
| Wasser | ad 100,00 |

Die unter (a) und (c) genannten Bestandteile werden jeweils unter Rühren auf 75° C erwärmt. Sodann werden die Bestandteile (a) zu (c) gegeben. Die Mischung wird auf 35° C abgekühlt. Komponente (b) wird unter Rühren zur Mischung aus (a) und (c) gegeben.

### Beispiel 5

| Gesichtswasser I | |
|---|---|
| | Gew.-% |
| Ethanol | 10,00 |
| DMC | 0,70 |
| TML | 1,00 |
| Wasser | ad 100,00 |

### Beispiel 6

| Gesichtswasser II | |
|---|---|
| | Gew.-% |
| Ethanol | 10,00 |
| DMC | 0,80 |
| Wasser | ad 100,00 |

### Beispiel 7

| Hautreinigungs-Gel III | |
|---|---|
| (a) | Gew.-% |
| 1,3-Butylenglycol | 2,00 |
| Hydroxyethylcellulose (z.B. Tylose 4000, Hoechst) | 0,50 |

| (b) | |
|---|---|
| Ethylalkohol | 60,00 |
| PEG-40-Hydriertes Ricinusöl | 2,00 |
| DML | 0,90 |

| (c) | |
|---|---|
| Wasser | ad 100,00 |

Die unter (a) genannten Bestandteile werden dispergiert, Wasser (c) wird zugegeben, bei Raumtemperatur quellen gelassen, eine Lösung der unter (b) genannten Bestandteile wird nach ca. 15 Minuten zugegeben. Die entstanden Mischung wird homogenisiert und kann abgefüllt werden.

### Beispiel 8

| Reinigungs-Emulsion III | |
|---|---|
| (a) | Gew.-% |
| Tricetearethphosphat | 0,30 |
| Octyldodecanol | 2,00 |
| C₁₂₋₁₅-Alkyl-Benzoate | 2,00 |
| DML | 0,90 |
| C₁₂₋₃₀-Alkylacrylate | 0,15 |

| (b) | |
|---|---|
| Ethylalkohol | 10,00 |

| (c) | |
|---|---|
| NaOH | 0,05 |
| Wasser | ad 100,00 |

Die unter (a) und (c) genannten Bestandteile werden jeweils unter Rühren auf 75° C erwärmt. Sodann werden die Bestandteile (a) zu (c) gegeben. Die Mischung wird auf 35° C abgekühlt. Komponente (b) wird unter Rühren zur Mischung aus (a) und (c) gegeben.

## Patentansprüche

1. Verwendung von Monocarbonsäureestern des Diglycerins zur Herstellung von Zubereitungen gegen unreine Haut und/oder gegen *Propionibacterium acnes*.

2. Verwendung von Monocarbonsäureestern des Diglycerins zur Herstellung von Zubereitungen gegen unreine Haut und/oder gegen *Propionibacterium acnes* in kosmetischen Zubereitungen.

3. Verwendung nach Anspruch 1 oder 2, dadurch gekennzeichnet, daß das Diglycerin in 1-Stellung mit Carbonsäuren verestert vorliegt.

4. Verwendung nach Anspruch 1 oder 2, dadurch gekennzeichnet, daß die Monocarbonsäureester des Diglycerins Monocarbonsäuremonoester darstellen und durch folgende Strukturen gekennzeichnet sind: 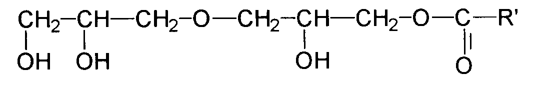 wobei R' einen Kohlenwasserstoffrest, vorteilhaft einen verzweigten oder unverzweigten Alkyl- oder Alkenylrest von 5 bis 17 C-Atomen darstellt.

5. Verwendung nach Anspruch 1 oder 2, dadurch gekennzeichnet, daß R' gewählt wird aus der Gruppe der unverzweigten Alkylreste mit ungeraden C-Zahlen, insbesondere mit 9, 11 und 13 C-Atomen, bevorzugt aus der Gruppe
| | | |
|---|---|---|
| Diglycerinmonocaprinat | (DMC) | R' = 9 |
| Diglycerinmonolaurat | (DML) | R' = 11 |
gewählt wird.

6. Verwendung nach Anspruch 1 oder 2, dadurch gekennzeichnet, daß der oder die Monocarbonsäureester des Diglycerins in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt oder vorliegen.

7. Verwendung nach Anspruch 1 oder 2, dadurch gekennzeichnet, daß sie in Form von Gesichtswässern, flüssigen Reinigungsemulsionen oder -lotionen, Stiften, hautklärenden Cremes oder Lotionen oder Tinkturen vorliegen.

## Claims

1. Use of monocarboxylic acid eaters of diglycerol for the preparation of formulations against blemished skin and/or against *Propionibacterium acnes*.

2. Use of monocarboxylic acid esters of diglycerol for the preparation of formulations against blemished skin and/or against *Propionibacterium acnes* in cosmetic formulations,

3. Use according to Claim 1 or 2, characterized in that the diglycerol is esterified with carboxylic acids in the 1-position.

4. Use according to Claim 1 or 2, characterized in that the diglycerol monocarboxylic acid eaters are monocarboxylic acid monoesters and are characterized by the following structures: 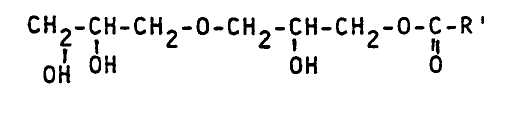 wherein R' is a hydrocarbon radical, advantageously a branched or unbranched alkyl or alkenyl radical having 5 to 17 C atoms.

5. Use according to Claim 1 or 2, characterized in that R' is chosen from the group consisting of unbranched alkyl radicals having odd C numbers, in particular with 9, 11 and 13 C atoms, preferably from the group consisting of
| | | |
|---|---|---|
| diglycerol monocaprate | (DMC) | R' = 9 |
| diglycerol monolaurate | (DML) | R' = 11. |

6. Use according to Claim 1 or 2, characterized in that the monocarboxylic acid ester or eaters of diglycerol is or are present in concentrations of 0.01 - 10.00 % by weight, preferably 0.05 - 5.00 % by weight, particularly preferably 0.1 - 3.00 % by weight, in each case based on the total weight of the composition.

7. Use according to Claim 1 or 2, characterized in that they are in the form of face lotions, liquid cleansing emulsions or lotions, sticks, skin-clarifying creams or lotions or tinctures.

## Revendications

1. Utilisation d'esters d'acides monocarboxyliques du diglycérol pour la fabrication de préparations contre les impuretés de la peau et/ou contre *Propionibacterium acnes*.

2. Utilisation d'esters d'acides monocarboxyliques du diglycérol pour la fabrication de préparations contre les impuretés de la peau et/ou contre *Propionibacterium acnes*, dans des préparations cosmétiques.

3. Utilisation selon la revendication 1 ou 2, caractérisée en ce que le diglycérol est estérifié en position 1 par des acides carboxyliques.

4. Utilisation selon la revendication 1 ou 2, caractérisée en ce que les esters d'acides monocarboxyliques du diglycérol représentent des monoesters d'acides monocarboxyliques et sont caractérisés par la structure suivante: 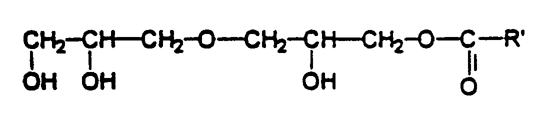 R' représentant un radical hydrocarboné, avantageusement un radical alkyle ou alcényle ramifié ou non ramifié ayant de 5 à 17 atomes de carbone.

5. Utilisation selon la revendication 1 ou 2, caractérisée en ce que R' est choisi dans le groupe des radicaux alkyle non ramifiés à nombre impair d'atomes de carbone, en particulier à 9, 11 et 13 atomes de carbone, et de préférence est choisi dans le groupe constitué par
| | | |
|---|---|---|
| le monocaprate de diglycérol | (DMC) | R' = 9, |
| le monolaurate de diglycérol | (DML) | R' = 11. |

6. Utilisation selon la revendication 1 ou 2, caractérisée en ce que le ou les esters d'acides monocarboxyliques du diglycérol se trouve ou se trouvent à des concentrations de 0,01-10,00% en poids, de préférence de 0,05-5,00% en poids, de façon particulièrement préférée de 0,1-3,00% en poids, dans chaque cas par rapport au poids total de la composition.

7. Utilisation selon la revendication 1 ou 2, caractérisée en ce que les préparations se trouvent sous forme de lotions faciales, d'émulsions ou de lotions liquides de nettoyage, de bâtons, de crèmes clarifiantes pour la peau ou de lotions ou de teintures.
